# EUROPEAN PATENT APPLICATION

(11) **EP 1 230 924 A1**
(43) Date of publication of application: **14.08.2002**
(21) Application number: 00969830.9
(22) Date of filing: 18.10.2000
(51) Int. Cl.: A61K 31/593, A61K 7/00, A61P 17/00

(54) **REMEDIES FOR PIGMENTATION AND MELANOCYTE PROLIFERATION INHIBITORS**

(30) Priority: 19.10.1999 JP 29664999
(71) Applicant: Nakayama, Juichiro, Fukuoka-shi, Fukuoka 810-0024 (JP)
(72) Inventor: Nakayama, Juichiro, Fukuoka-shi, Fukuoka 810-0024 (JP)
(74) Representative: VOSSIUS & PARTNER
(86) International application number: JP0007206
(87) International publication number: WO0128565

(57) **Abstract**

The present invention aims to provide an effective medicine for excessive proliferation of melanocytes or pigmentation.

The present invention provides therapeutic agents for pigmentation and melanocyte growth inhibitory agents containing a vitamin D derivative as an active ingredient.

## Description

### TECHNICAL FIELD

The present invention relates to therapeutic agents for pigmentation and melanocyte growth inhibitory agents containing a vitamin D derivative as an active ingredient.

### BACKGROUND ART

Generally, the color of the human skin or mucous membrane depends on melanin, oxidized or reduced hemoglobin, carotene and the like to show dark brown, white, yellow, red, dark blue and other tones. Among others, melanin is an important factor for determining the skin color and responsible for pigmentation or other conditions due to an abnormality in the production level thereof or the number or function of melanin-producing cells, i.e., melanocytes. Melanocytes are pigment-producing cells intervening between basal cells, and the thus produced melanin pigments are transferred from their cell processes into adjacent basal cells or prickle cells.

Pigmentation disorders of the skin or mucous membrane include pigmentation after sunburn, cafe-au-lait spots, freckles, chloasma, melanoderma, senile pigment macula, floriform pigmentation, solar keratosis, pigmented nevus, Ota's nevus, berloque dermatitis, etc. The mechanism by which these pigmentation disorders occur has been unknown in many aspects, but may be generally explained by abnormal deposition of melanin in the skin or mucous membrane due to internal diseases such as endocrine or metabolic disorders, hereditary predisposition, pregnancy, stimulation by UV rays from sunlight, photodynamic or other skin stimulation, progressed allergic contact dermatitis, etc.

Internal diseases associated with pigmentation include endocrine or metabolic disorders such as Addison's disease, Cushing's disease or syndrome, hyperpituitarism, ectopic ACTH producing tumors, hyperthyroidism, adrenogenital syndrome and Nelson's syndrome; collagen diseases such as systemic scleroderma; malignant visceral tumors linked to acanthosis nigricans, etc. Pigmentation is also observed in patients of hereditary or congenital diseases such as von Recklinghausen's disease, Albright's syndrome, multiple lentigines syndrome, Peutz-Jeghers' syndrome and neurocutaneous melanosis or medicated with anticancer drugs (5-FU, cyclophosphamide, bleomycin, methotrexate, busulphan, etc.), ACTH, hydantoin drugs, oral contraceptives, etc.

Conventional therapies for these pigmentation disorders include oral administration of vitamin C, glutathione, tranexamic acid (JPA No. 93519/1989) or the like; external application of vitamin C, cysteine, hydroquinone or the like; and laser therapy. However, administration of vitamin C, glutathione, cysteine or tranexamic acid is very slow to produce an effect, and administration of hydroquinone or laser therapy strongly irritates the skin or mucous membrane and involves the risk of recurrence.

JPA No. 145211/1988 discloses the use of a cosmetic containing a vitamin D derivative for treating "spots" or "dull skin", but its actual effect is not shown and the development of effective therapy for pigmentation has been anticipated for some time.

### DISCLOSURE OF THE INVENTION

As described above, conventional therapies or therapeutic agents were not sufficiently effective for pigmentation disorders of the skin or mucous membrane. The present invention aims to provide an effective medicine for these pigmentation disorders.

As a result of careful studies on therapeutic agents for pigmentation, we accomplished the present invention on the basis of the finding that vitamin D derivatives have a melanocyte growth inhibitory effect and are effective for the improvement/treatment of pigmentation.

Accordingly, the present invention provides therapeutic agents for pigmentation and melanocyte growth inhibitory agents comprising a vitamin D derivative as an active ingredient.

Preferably, therapeutic agents for pigmentation and melanocyte growth inhibitory agents of the present invention contain as an active ingredient a compound represented by Formula (I): 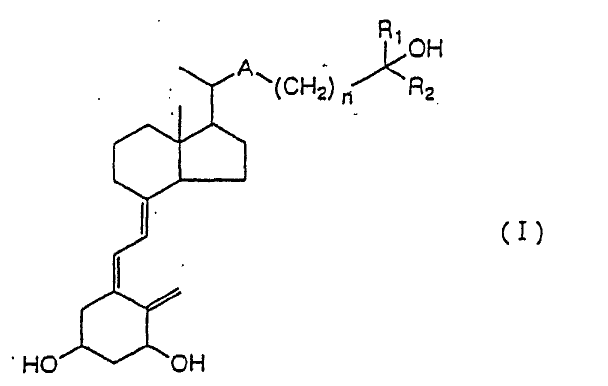 wherein R₁ and R₂ are the same or different and each represent a lower alkyl group containing 1 to 4 carbon atoms; A represents -CH₂- or an oxygen or sulfur atom; and n represents 2 or 3.

Therapeutic agents for pigmentation and melanocyte growth inhibitory agents of the present invention may contain as an active ingredient a compound represented by Formula (II): 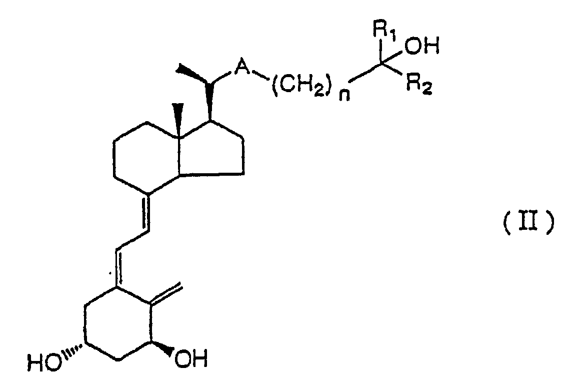 wherein R₁ and R₂ are the same or different and each represent a lower alkyl group containing 1 to 4 carbon atoms; A represents -CH₂- or an oxygen or sulfur atom; and n represents 2 or 3;
or Formula (III): 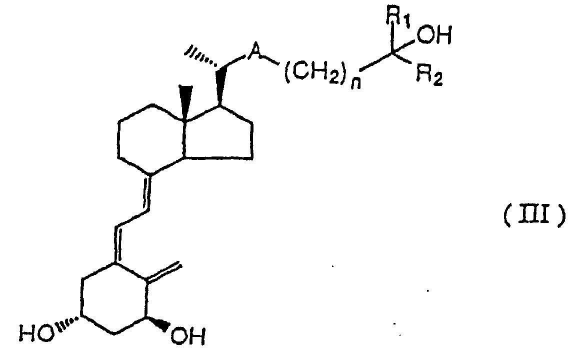 wherein R₁ and R₂ are the same or different and each represent a lower alkyl group containing 1 to 4 carbon atoms; A represents -CH₂- or an oxygen or sulfur atom; and n represents 2 or 3.

In Formula (I), (II) or (III) above, R₁ and R₂ are preferably the same or different and each represent a methyl or ethyl group, more preferably R₁ and R₂ are the same and represent a methyl or ethyl group.

Preferably, therapeutic agents for pigmentation and melanocyte growth inhibitory agents of the present invention contain as an active ingredient a compound represented by Formula (IV):

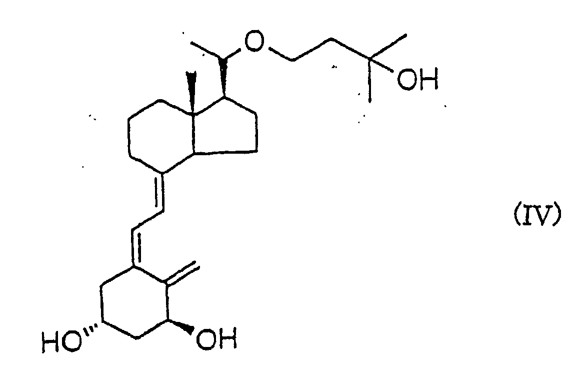

### BRIEF EXPLANATION OF THE DRAWINGS

Fig. 1 is a graph showing the inhibitory effect of 22-oxacalcitriol (OCT) and calcitriol on the proliferation of cultured human melanocytes;
Fig. 2 is a graph showing the inhibitory effect of 22-oxacalcitriol (OCT) and calcitriol on the proliferation of cultured human keratinocytes; and
Fig. 3 is a graph showing the inhibitory effect of 22-oxacalcitriol (OCT) and calcitriol on ³[H]-TdR uptake by cultured human melanocytes.

### PREFERRED MODE FOR CARRYING OUT THE INVENTION

Preferred vitamin D derivatives in the present invention are *in vivo* physiologically active vitamin D derivatives such as 1α,25-dihydroxyvitamin D₃ (calcitriol), 1α-hydroxyvitamin D₃, 25-hydroxyvitamin D₃, 22-oxacalcitriol (OCT), 22-thiacalcitriol and 5,6-trans-25-hydroxyvitamin D₃.

Particularly, a compound represented by Formula (I): 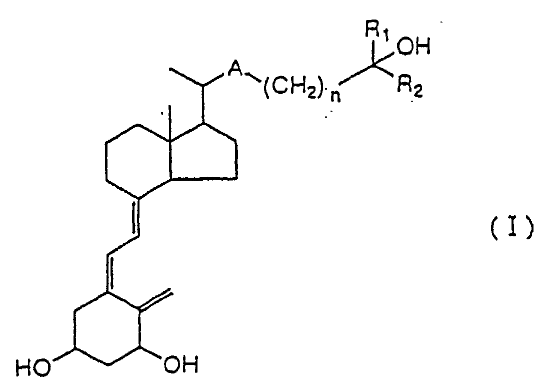 wherein R₁ and R₂ are the same or different and each represent a lower alkyl group containing 1 to 4 carbon atoms; A represents -CH₂- or an oxygen or sulfur atom; and n represents 2 or 3
is preferably contained as an active ingredient.

In the present invention, the lower alkyl group containing 1 to 4 carbon atoms may be straight or branched, such as methyl, ethyl, n-propyl, i-propyl, n-butyl, i-butyl, s-butyl and t-butyl groups, preferably methyl or ethyl. In the present invention, R₁ and R₂ may be the same or different, but preferably the same. Specific examples include compounds of Formula (I), (II) or (III) wherein R₁ and R₂ are the same or different and each represent a methyl or ethyl group, or compounds of Formula (I), (II) or (III) wherein R₁ and R₂ are identical and represent a methyl or ethyl group.

Vitamin D derivatives of the present invention of the above Formula in which A represents an oxygen atom are described in, for example, JPB No. 74656/1991, International Publication WO90/09991 etc. Those in which A represents a sulfur atom are described in International Publication WO94/14766 and the abstracts of oral reports in the 15th Medicinal Chemistry Symposium (published by the pharmaceutical chemical group of the Pharmaceutical Society of Japan, Nov. 1, 1994, pp. 97-98).

Generally, vitamin Ds are known to show a wide range of physiological activities such as calcium metabolism regulation, growth inhibition and differentiation induction of tumor cells and immunoregulation. However, no reports suggest that they inhibit proliferation of melanocytes or are effective for treating pigmentation.

Therapeutic agents for pigmentation or melanocyte growth inhibitory agents of the present invention can be in dosage forms for external application such as creams, ointments and lotions prepared by ordinary formulation processes of vitamin D or non-invasive dosage forms such as solutions including injections based on aqueous solvents or can be formulated for oral administration, but preferably for external application.

Preferably, pharmaceutical agents of the present invention are topically administered in the form of an external formulation, but may also be systemically administered in the form of an oral formulation or injection in some cases.

The dose of vitamin D derivatives of the present invention depends on the age, sex, condition and other factors, but normally ranges from 0.01 to 10000 µg, preferably from 0.1 to 1000 µg, more preferably from 1 to 100 µg per individual daily.

Compounds of the present invention can be synthesized by, for example, the process described from column 4, line 36 to column 13, line 10 of JPA No. 268894/1996 but can also be synthesized by other known processes.

The present application claims priority based on Japanese Patent Application No. 296649/1999, the disclosure of which is wholly incorporated herein as reference.

### EXAMPLES

The following examples further illustrate the present invention without, however, limiting the invention thereto.

In the following examples, vitamin D derivatives were examined for growth inhibitory effect on cultured human melanocytes and keratinocytes.

The melanocytes, keratinocytes and vitamin D derivatives used in each example are as follows.

Melanocytes: normal human epidermal melanocytes (NHEM:KM-4009, Kurabo Industries).

Keratinocytes: normal human epidermal keratinocytes (HEKn:C-001-5C, Kurabo Industries).

Vitamin D derivatives: 22-oxacalcitriol (OCT) and calcitriol (1α,25-dihydroxyvitamin D₃).

### Example 1

Melanocytes were cultured at 2 x 10⁵ cells/well in MGM3 (Melanocyte Growth Medium 3, Takara Shuzo) used as a culture medium. Keratinocytes were cultured at 1 x 10⁵ cells/well in KGM2 (Keratinocyte Growth Medium 2, Takara Shuzo) used as a culture medium. To each well was added 22-oxacalcitriol or calcitriol at a final concentration of 10⁻⁷ M and cultivation was continued. On days 3 and 6 after the addition, the number of cells was determined by a Coulter Counter.

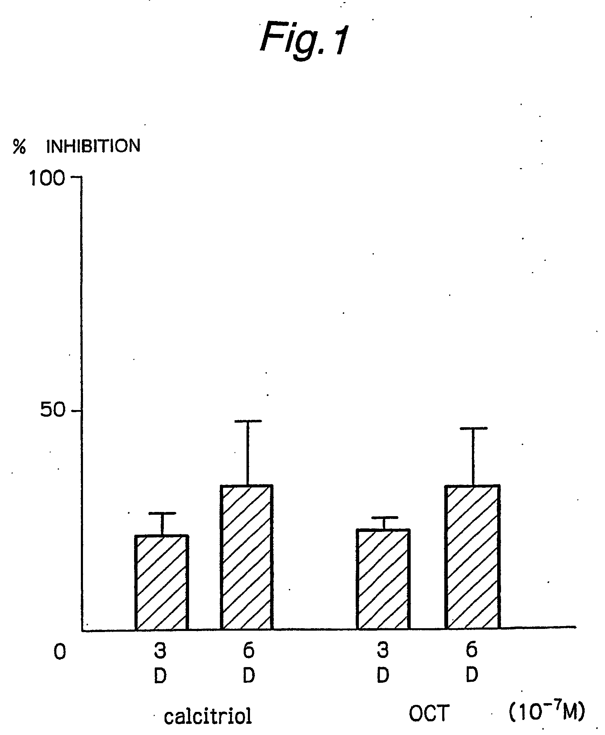

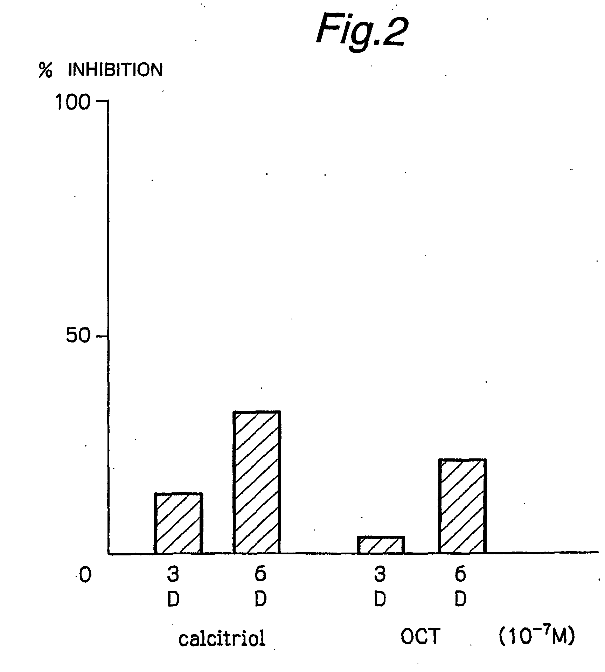

The results are shown in Figs. 1 and 2. Each datum shows the average of 5 experiments. Bars in Fig. 1 represent inhibition ±S.E. As shown in Fig. 1, both calcitriol and 22-oxacalcitriol groups showed an inhibitory effect of 30% or more against the proliferation of melanocytes on day 6. As shown in Fig. 2, calcitriol group and 22-oxacalcitriol group also showed inhibition of 40% and 25%, respectively, against keratinocytes on day 6.

### Example 2

Human melanocytes were cultured at 5 x 10⁵ cells/well in MGM3 used as a culture medium, and 22-oxacalcitriol or calcitriol was added to each well at a final concentration of 10⁻⁷ M to determine tritiated thymidine (³[H]-TdR) uptake by cells in a liquid scintillation counter on days 1, 3 and 5 after the addition.

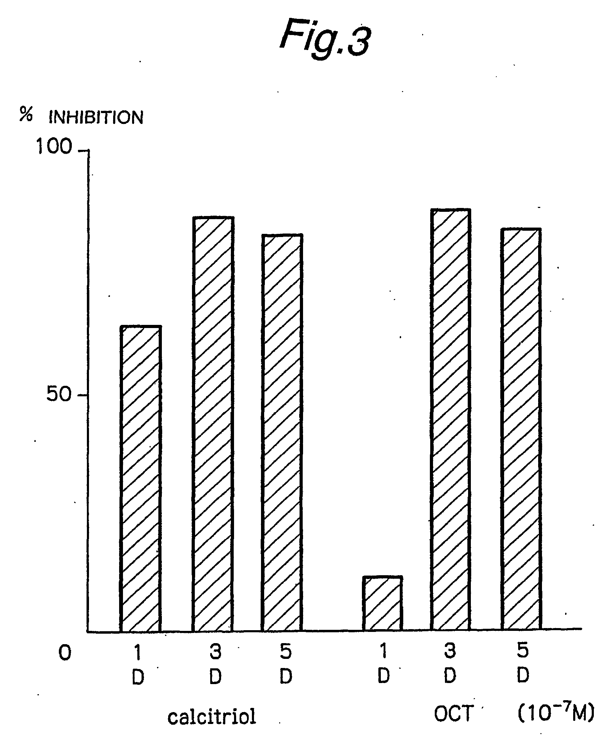

The results are shown in Fig. 3. On day 1, calcitriol group showed a 50% decrease in uptake. On day 3, both calcitriol and 22-oxacalcitriol groups showed a decrease of 80% or more in uptake.

As shown in Example 1, both 22-oxacalcitriol and calcitriol apparently inhibited proliferation of melanocytes at a concentration of 10⁻⁷ M. This inhibitory effect was also shown by a clear decrease in tritiated thymidine uptake in Example 2.

Details of the melanocyte proliferation inhibition mechanism of vitamin D derivatives such as 22-oxacalcitriol and calcitriol are unknown. However, it is thought that the inhibition of melanocyte proliferation and the decrease in the number of melanocytes may decrease melanin production and lead to an improvement/therapy of pigmentation caused by increased melanin levels.

For example, an increase of melanocytes in the epidermal basement membrane has been reported in pigmentation disorders such as cafe-au-lait spots found in Recklinghausen's disease. Therefore, if a therapeutic agent containing a vitamin D derivative such as 22-oxacalcitriol is applied to these pigmentation disorders, it is expected to contribute to the improvement of pigmentation by inhibiting proliferation of melanocytes.

### INDUSTRIAL APPLICABILITY

As apparent from the foregoing description, vitamin D derivatives of the present invention have an excellent melanocyte growth inhibitory effect so that they are expected to be useful as melanocyte growth inhibitory agents and therapeutic agents for pigmentation.

## Claims

1. A therapeutic agent for pigmentation comprising a vitamin D derivative as an active ingredient.

2. The therapeutic agent of Claim 1, wherein the active ingredient is a compound represented by Formula (I): 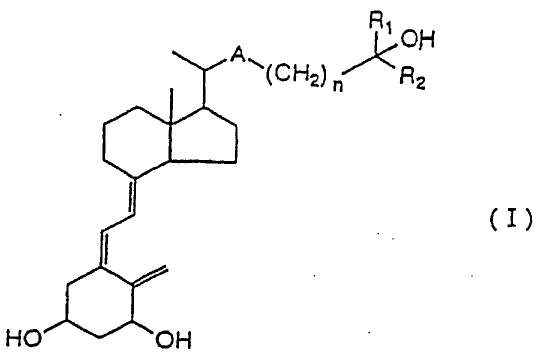 wherein R₁ and R₂ are the same or different and each represent a lower alkyl group containing 1 to 4 carbon atoms; A represents -CH₂- or an oxygen or sulfur atom; and n represents 2 or 3.

3. The therapeutic agent of Claim 1 or 2, wherein the active ingredient is a compound represented by Formula (II): 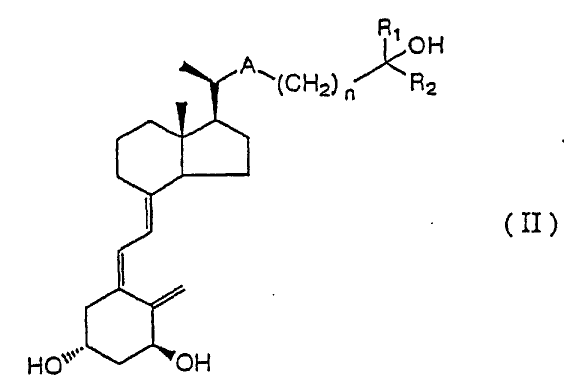 wherein R₁ and R₂ are the same or different and each represent a lower alkyl group containing 1 to 4 carbon atoms; A represents -CH₂- or an oxygen or sulfur atom; and n represents 2 or 3.

4. The therapeutic agent of Claim 1 or 2, wherein the active ingredient is a compound represented by Formula (III): 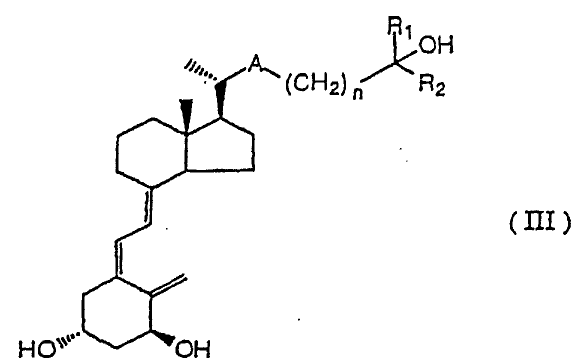 wherein R₁ and R₂ are the same or different and each represent a lower alkyl group containing 1 to 4 carbon atoms; A represents -CH₂- or an oxygen or sulfur atom; and n represents 2 or 3.

5. The therapeutic agent of any one of Claims 1 to 4, wherein R₁ and R₂ are the same or different and each represent a methyl or ethyl group.

6. The therapeutic agent of any one of Claims 1 to 4, wherein R₁ and R₂ are the same and represent a methyl or ethyl group.

7. The therapeutic agent of any one of Claims 1 to 4, wherein the active ingredient is a compound represented by Formula (IV).

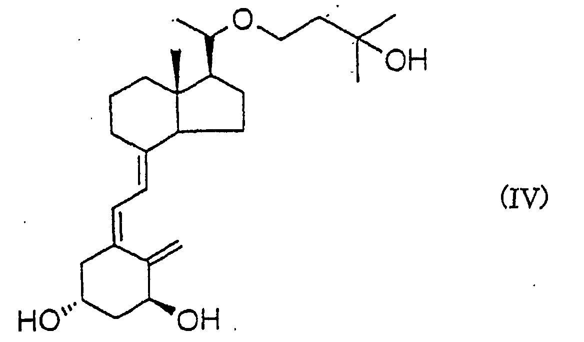

8. A melanocyte growth inhibitory agent comprising a vitamin D derivative as an active ingredient.

9. The growth inhibitory agent of Claim 8, wherein the active ingredient is a compound represented by Formula (I): 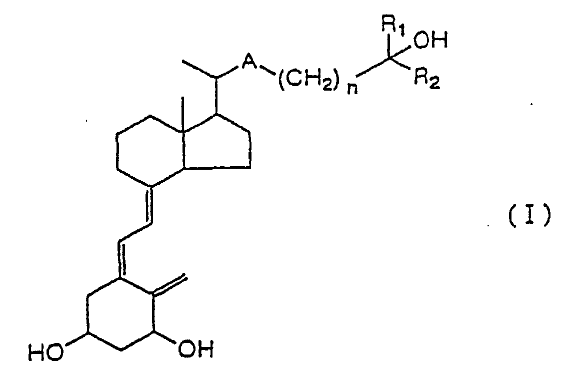 wherein R₁ and R₂ are the same or different and each represent a lower alkyl group containing 1 to 4 carbon atoms; A represents -CH₂- or an oxygen or sulfur atom; and n represents 2 or 3.

10. The growth inhibitory agent of Claim 8 or 9, wherein the active ingredient is a compound represented by Formula (II): 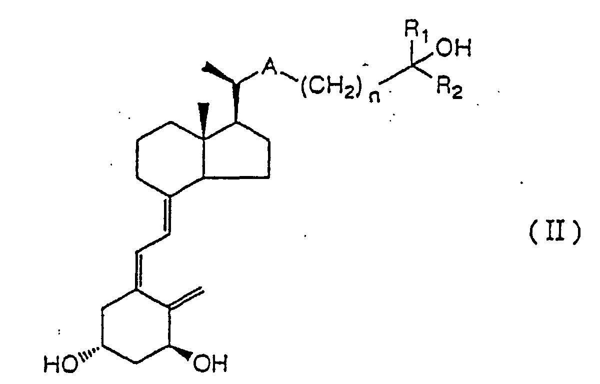 wherein R₁ and R₂ are the same or different and each represent a lower alkyl group containing 1 to 4 carbon atoms, A represents -CH₂- or an oxygen or sulfur atom, and n represents 2 or 3.

11. The growth inhibitory agent of Claim 8 or 9, wherein the active ingredient is a compound represented by Formula (III): 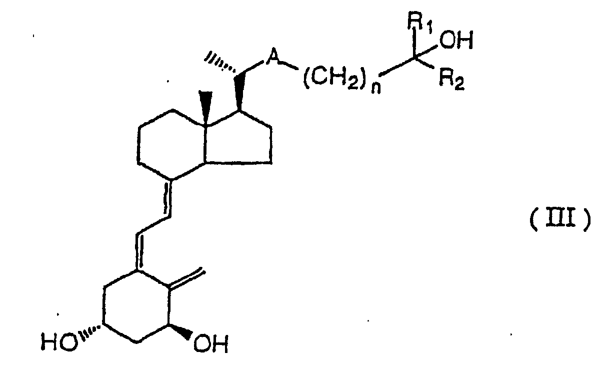 wherein R₁ and R₂ are the same or different and each represent a lower alkyl group containing 1 to 4 carbon atoms, A represents -CH₂- or an oxygen or sulfur atom, and n represents 2 or 3.

12. The growth inhibitory agent of any one of Claims 8 to 11, wherein R₁ and R₂ are the same or different and each represent a methyl or ethyl group.

13. The growth inhibitory agent of any one of Claims 8 to 11, wherein R₁ and R₂ are the same and represent a methyl or ethyl group.

14. The melanocyte growth inhibitory agent of any one of Claims 8 to 11, wherein the active ingredient is a compound represented by Formula (IV).
